# EUROPEAN PATENT APPLICATION

(11) **EP 4 431 072 A1**
(43) Date of publication of application: **18.09.2024**
(21) Application number: 23162472.7
(22) Date of filing: 16.03.2023
(51) Int. Cl.: A61F 13/56

(54) **SANITARY NAPKIN**

(71) Applicant: Ontex BV, 9255 Buggenhout (BE); Ontex Group NV, 9320 Erembodegem (BE)
(72) Inventor: VAN KETS, Lies, 9900 Eeklo (BE); STAELENS, Eva, 9900 Eeklo (BE)
(74) Representative: Saurat, Thibault

(57) **Abstract**

The invention pertains to sanitary napkin (10) comprising :
- a topsheet (14),
- a backsheet (16),
- an absorbent core (12) arranged between said topsheet (14) and backsheet (16),
- two wings (24),
the sanitary napkin (10) extending in longitudinal, transversal and vertical directions (L,T,V), the sanitary napkin (1) being divided with respect to the longitudinal direction, into a front portion (F), a rear portion (R) and a central portion (C) arranged between said front and rear portions, the central portion (C) being defined by the length of the wings (24) taken in the point in which they extend from the longitudinal sides (32) of the sanitary napkin (10) in a direction parallel to the longitudinal axis (y-y),
wherein a layer of adhesive (20) is arranged on the side of the backsheet (16) opposite to the absorbent core (12).

According to the invention, said layer of adhesive (20) is arranged on the backsheet (16) in a discontinuous pattern, said pattern of adhesive comprising:
- a first area of adhesive (40) arranged in the front portion (F),
- a second area of adhesive (42) arranged in the rear portion (R);
- a third area of adhesive (45) being arranged on the wings (24);
wherein said pattern of adhesive comprises an adhesive-free area (46) between the first area of adhesive (40) and the second area of adhesive (42), said adhesive-free area (46) longitudinally extending on a length that is greater than or equal to the length of the third area of adhesive (45) with respect to the longitudinal direction (L).

## Description

### TECHNICAL FIELD

The disclosure pertains to the technical field of absorbent articles for hygiene products and the packaging of such absorbent articles. In particular, the present disclosure relates to individually packaged absorbent articles selected from sanitary napkins, adult incontinence pads or panty liners, which are configured to collect and contain blood, menses, urine, vaginal fluids or avoid leakage..

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged inbetween said topsheet and backsheet. These articles are configured to be fastened onto an undergarment and comprise a layer of adhesive arranged on one side of the backsheet so that the article can be secured onto said garment.

For example, document WO03/072004 describes such sanitary napkin where the two longitudinally oriented strips are applied on the entire length of the backsheet to better secure the sanitary napkin to the garment.

There is still a need to improve the environmental impact of these absorbent articles and offer a solution that involves less material without degrading the performance of these absorbent articles.

The invention thereto aims to provide an environmentally friendly packaging for absorbent articles selected from sanitary napkins, adult incontinence pads or pantyliners.

### SUMMARY OF THE INVENTION

The present invention relates to a sanitary napkin comprising :
- a topsheet,
- a backsheet,
- an absorbent core arranged between said topsheet and backsheet,
- two wings,

the sanitary napkin extending in longitudinal, transversal and vertical directions, preferably the sanitary napkin has a longitudinal central axis, a pair of transverse sides and a pair of longitudinal sides extending therebetween, preferably the sanitary napkin has a front edge and a rear edge positioned on opposite apexes of said transverse sides, preferably the wings extend transversely beyond the longitudinal sides of the sanitary napkin, e.g. when laid flat on a surface in a unfolded configuration,
the sanitary napkin being divided with respect to the longitudinal direction, into a front portion, a rear portion and a central portion arranged between said front and rear portions, the central portion being defined by the length of the foldable wings taken in the point in which they extend from the longitudinal sides of the sanitary napkin in a direction parallel to the longitudinal axis,
wherein a layer of adhesive is arranged on the side of the backsheet opposite to the absorbent core, or in other words wherein a layer of adhesive is arranged on one side of the backsheet, the backsheet being arranged between the layer of adhesive and the absorbent core.According to the present invention, said layer of adhesive is arranged on the backsheet in a discontinuous pattern, said pattern of adhesive comprising:
   - a first area of adhesive arranged in the front portion,
   - a second area of adhesive arranged in the rear portion,
   - a third area of adhesive being arranged on the wings,
wherein said pattern of adhesive comprises an adhesive-free area between the first area of adhesive and the second area of adhesive where no adhesive is present, said adhesive-free area longitudinally extending on a length that is greater than or equal to the length of the third area of adhesive with respect to the longitudinal direction.

Said adhesive-free area between the first and second areas of adhesive can also be called a gap or an adhesive-free gap or an adhesive-free zone or an adhesive-free region.

Comprising a gap or adhesive-free area extending in the central portion more or less, between the first area and the second area, saves on raw material and enables to fold the wings towards the backsheet since no adhesive is present in the central portion. Having the wings folded onto the backsheet enables to have a sanitary napkin with only one single sheet of release paper to covering both adhesive on the backsheet and on the wings, said single sheet having lesser dimensions than the backsheet in length and in width..

According to an embodiment, said adhesive-free area extends transversally between the two wings.

According to an embodiment, said adhesive-free area extends longitudinally between the first area of adhesive and the second area of adhesive on a length that is greater than or equal to the length of the third area of adhesive with respect to the longitudinal direction and said adhesive-free area extends transversally between the two wings.

According to an embodiment, the first area of adhesive and second area of adhesive are aligned on one same longitudinal axis.

According to an embodiment, the third area of adhesive is longitudinally skewed from the first and second areas of adhesive when projected on the longitudinal axis.

According to an embodiment, the wings are folded onto the backsheet or topsheet, the first, second and third areas of adhesive are aligned with respect to the longitudinal axis.

According to an embodiment, the first, second and/or third areas of adhesive comprise strips of adhesive, said strips extending transversally and/or longitudinally.

According to an embodiment, a first release paper and/or a pouch is arranged on one side of the layer of adhesive such that the layer of adhesive is arranged between the backsheet and the release paper and/or pouch.

According to an embodiment, a single release paper simultaneously covers all three areas of adhesive, first and second areas of adhesive being covered by a first side of the release paper and the third area of adhesive being covered by the opposite side of the release paper.

According to an embodiment, the sanitary napkin comprises a second release paper covering the third areas of adhesive, the wings being separated from one another by a gap with respect to the transversal direction T when folded over the backsheet. Optionnally, an additional layer of adhesive can be arranged between the first and second release papers within said gap.

All of these embodiments mentioned above can be taken individually or in combination.

The invention also relates to a method suitable for packaging a sanitary napkin for obtaining an assembly as described herein, the method comprising
- providing a sanitary napkin comprising a topsheet, backsheet and an absorbent core arranged in between,
- applying a layer of adhesive according to a discontinuous pattern of adhesive wherein said pattern of adhesive comprises:
- a first area of adhesive arranged in the front portion,
- a second area of adhesive arranged in the rear portion;
- a third area of adhesive being arranged on the wings;
wherein the pattern of adhesive comprises an adhesive-free area between the first area of adhesive and the second area of adhesive where no adhesive is present, said adhesive-free area extending on a greater length than the third area of adhesive with respect to the longitudinal direction.

According to an embodiment, said adhesive-free area extends transversally between the two wings.

According to an embodiment, said adhesive-free area extends longitudinally between the first area of adhesive and the second area of adhesive on a length that is greater than or equal to the length of the third area of adhesive with respect to the longitudinal direction and said adhesive-free area extends transversally between the two wings.

All of these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**Fig. 1** illustrates a schematic transversal cross section of the central portion of an absorbent article without a release paper;
**Fig. 2** shows a schematic bottom view of a partial absorbent article in a unfolded configuration and showing the pattern of adhesive according to an embodiment;
**Fig. 3A** depicts a schematic top view of a partial absorbent article in a configuration where the wings have been folded and showing the pattern of adhesive according to an embodiment;
**Fig. 3B** illustrates a schematic view looking in the longitudinal direction of the absorbent article illustrated in FIG. 3A.
**Fig. 4A** depicts a schematic top view of a partial absorbent article in a configuration where the wings have been folded and showing the pattern of adhesive according to another embodiment;
**Fig. 4B** illustrates a schematic view looking in the longitudinal direction of the absorbent article illustrated in FIG. 4A.
**Fig. 5** shows a schematic top view of a partial absorbent article in a configuration where the wings have been folded and showing the pattern of adhesive according to another embodiment;
**Fig. 6** depicts a schematic top view of a partial absorbent article in a configuration where the wings have been folded and showing the pattern of adhesive according to another embodiment;
**Fig. 7** illustrates a perspective view of a wrapper enclosing a sanitary napkin.

### DETAILED DESCRIPTION OF THE INVENTION

The present invention concerns the packaging of individually packaged absorbent articles selected from sanitary napkins, adult incontinence pads or pantyliners.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
"Absorbent article" refers to devices that absorb and contain liquid, and more specifically, refers to devices that are placed against or in proximity to the body of the wearer to absorb and contain the various exudates discharged from the body. Absorbent articles preferably comprise a longitudinal axis defining a length and a transversal axis defining a width, said transversal axis being perpendicular to said longitudinal axis as well as a vertical axis defining a height or thickness. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.
"Front", "rear or back", and "crotch" end of the absorbent article as used herein typically refer for the "front" end of the article is that which is most proximal to the front of the subject when worn, the "rear or back" end of the article is that which is most proximal to the rear or back of the subject when worn, and the "crotch" portion of the core is the middle portion of the absorbent article between the "front" and "rear or back" portions.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

The term "adhesive" as used herein is intended to refer to any suitable hot melt, water or solvent borne adhesive that can be applied to a surface e.g. a paper layer or a film layer in the required pattern or network of adhesive areas. Accordingly, suitable adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the topsheet to the backsheet and non-structural adhesive or pressure sensitive adhesive or positioning adhesive for bonding the absorbent article to the wrapper and hence to the undergarment.

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene and polyurethane.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "backsheet" refers to a material forming the outer cover of the absorbent article. The backsheet prevents the exudates contained in the absorbent structure from wetting articles such as undergarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing through. Examples of breathable backsheet materials are porous polymeric films, nonwoven laminates of spunbond and meltblown layers and laminates of porous polymeric films and nonwoven materials. Alternatively, the backsheet can comprise, preferably consist of, a paper layer or a nonwoven layer for example with pantyliners where risks of leakage are not as great, hence such material can be used.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

For instance, the basis weight of the layer of cellulosic fibers is preferably measured according to the ISO 536 standard or any standard method. The amount of peeling agent or sealing agent on the wrapper can be measured by taking a sample of the wrapper where the peeling agent or the sealing agent is arranged, weighing said sample before and after removal of the peeling agent or sealing agent (in grams) the difference giving the weight of the peeling agent or sealing agent, and dividing the weight of peeling agent or sealing agent by the area of the sample (in m²) thereby obtaining the amount of peeling agent or sealing agent in gsm.

As used herein, the term "body-facing" side or surface means that side or surface of the absorbent article or component of said absorbent article which is intended to be disposed toward or placed adjacent to the body of the wearer during ordinary use, while the "garment-facing" side or surface is on the opposite side, and is intended to be disposed to face away from the wearer's body during ordinary use. Such garment-facing surface may be arranged to face toward or placed adjacent to the wearer's undergarments when the absorbent article is worn. In other words, the "body-facing side" of a component is the side proximal to the body of the wearer whereas "garment-facing side" of a component is the side proximal to the garment or undergarment of the wearer.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

As used herein, the term "cellulosic" or "cellulosic fibers" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

As used herein, the term "garment" means any type of apparel which may be worn. This includes incontinence products, surgical gowns, industrial workwear and coveralls, undergarments, pants, shirts, jackets and the like.

The term "graphic" includes, but is not limited to, any type of design, image, mark, figure, codes, words, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layer or webs have been formed from many processes such as for example, meltblowing processes, spunbonding processes, and bonded carded web processes.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood or hardwood, or a material comprising viscose. The material is typically lightweight and has absorbent properties.

By "substantially", it is meant at least the majority of the structure referred to.

Use of the term "substrate" includes, but is not limited to, woven or nonwoven webs, porous films, ink permeable films, cellulosic pulp, paper, composite structures, or the like.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "pouch", "wrapping sheet", "packaging sheet" or "wrapping construction" are equivalent to "wrapper".

The term "longitudinal", "transversal" and "vertical" as used herein are with respect to the sanitary napkin in an assembled state or in a disassembled or partially assembled state and in a folded, partially unfolded or unfolded configuration. In other word, the sanitary napkin defines the longitudinal, transversal and vertical directions L,T,V. The term "in the direction" as used herein means along that direction, e.g. in the longitudinal direction means along the longitudinal direction. The terms "top", "bottom", "upper" and "lower" are all in reference to said vertical direction. The term "longitudinally extending" in reference to an element, e.g. a patch of adhesive, as used herein means this element is elongated in the longitudinal direction, meaning that this element is extending more in the longitudinal direction than in the transversal or vertical directions. Similarly, the term "transversally extending" in reference to an element, e.g. a patch of adhesive, as used herein means this element is elongated in the transversal direction, meaning that this elements is extending more in the transversal direction than in the longitudinal or vertical directions. Similarly, the term "longitudinally and transversally extending" in reference to an element, e.g. a patch of adhesive, as used herein means this element is extending more in the longitudinal and transversal directions than in the vertical direction. As used herein "longitudinally elongated" means elongated in the longitudinal direction.

All length, width, and height or thickness can measured with a caliper preferably a micrometer caliper gauge by taking the largest distance separating two points along one direction as defined herein for a given element, e.g. the sanitary napkin in a folded state, the wrapper or the upper strip or the lower strip, or in other words, measuring the distance between two edges of said element along one direction.

The term "length", "width" and "thickness or height" as used herein are respectively in reference to the longitudinal, transversal and vertical direction as defined herein.

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments maybe combined with one or more other embodiments without departing from the intention of the disclosure and without generalization therefrom.

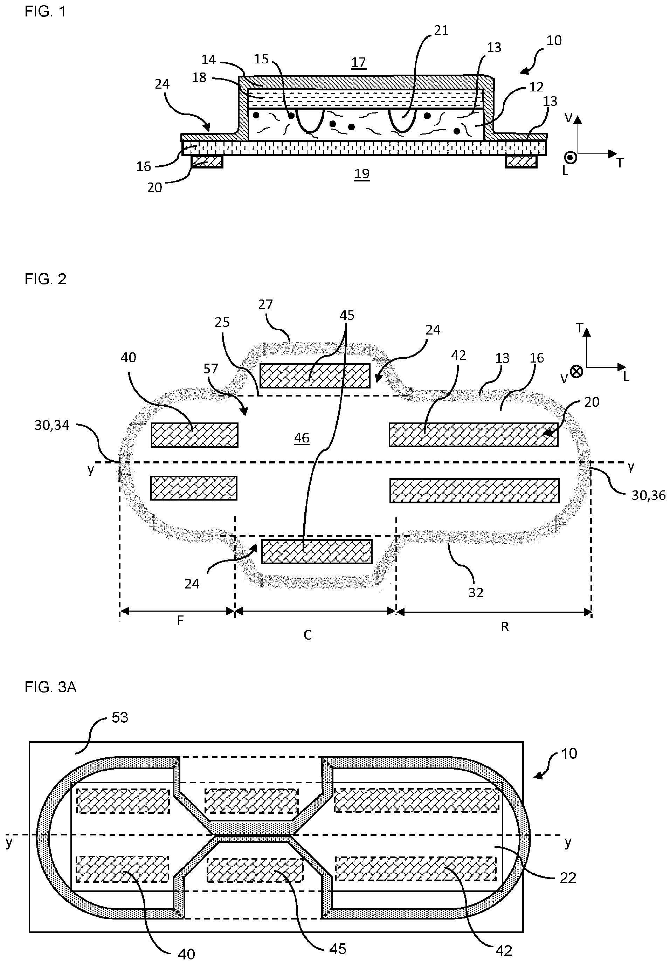

As illustrated in Fig.1, an absorbent article 10, for example a sanitary napkin 10, or a sanitary pad, or a sanitary towel, according to the present disclosure comprises an absorbent core 12 arranged between a liquid permeable topsheet 14 and a liquid impermeable backsheet 16. An optional acquisition distribution layer 18, or surge layer, also called ADL 18, can be positioned between said topsheet 14 and said absorbent core 12. The absorbent core 12 comprises absorbent material. The backsheet 16 is joined to the topsheet 14 with the absorbent core 12, and the optional ADL 18, being positioned and enclosed between the topsheet 14 and the backsheet 16. As illustrated in Fig. 1, the topsheet 14 and the backsheet 16 are associated with one another. The topsheet 14 and backsheet 16 are associated, or joined at the periphery of the sanitary napkin 10 as illustrated in FIG. 2 in bonding regions 13 by attachment means such as an adhesive, mechanical bonds, sonic bonds, thermal bonds, in other words, the bonding regions 13 define the outline or perimeter of the sanitary napkin 10. The topsheet 14 is arranged on the body-facing side 17 whereas the backsheet 16 is arranged on the garment-facing side 19 of the sanitary napkin 10. Optionally, the absorbent core 12 and/or the optional ADL 18, can comprise at least one channel 21 or at least one embossment 21. As illustrated in FIG. 1, the absorbent core 12 comprises two channels 21 or two embossments 21. The topsheet 14 and ADL 18 can be embossed as well.

The sanitary napkin 10 comprises on the garment-facing side 19 of the backsheet 16 at least one layer of adhesive 20 so that the sanitary napkin 10 can adhere onto a garment or undergarment, said at least one layer of adhesive 20 ensuring that the absorbent article 10 stays fastened and secured on the underwear. In order to maintain the structural integrity of the adhesive before being used, the sanitary napkin 10 comprises a release paper 22 corresponding to a peelable layer destined to protect the at least one layer of adhesive 20. The release paper 22 is preferably a siliconized paper acting as a release liner. In other words, the release paper 22 comprises a paper layer comprising cellulosic fibers, meaning paper, and a peeling layer or coating comprising a peeling agent selected from silicon resin series, fluororesin series, octadecilisocyanate series. In other words, the release paper 22 is a paper layer coated with a silicone derivative. According to an embodiment, the peeling layer comprises a basis weight comprised between 0,3 gsm and 3 gsm, preferably between 0,5 gsm and 1 gsm and the paper layer comprises a basis weight comprised between 10 gsm and 40 gsm. This release paper 22 is recyclable. The adhesive can be applied in bloc and/or in stripes and/or in dots and/or in swirls. The at least one layer of adhesive 20 is applied in a pattern that is described hereunder.

A sanitary napkin 10 is illustrated in FIG. 2 in an unfolded configuration, e.g. laid flat on a surface. The sanitary napkin 10 extends in the longitudinal, transversal and vertical directions L, T, V as illustrated in FIG. 1 and 2, thereby defining a length in the longitudinal direction L, a width W in the transversal direction T and a thickness or height in the vertical direction V. In an unfolded configuration as shown in FIG. 2, the length of the sanitary napkin 10 is greater than the width.

The sanitary napkin 10 comprises a topsheet 14, a backsheet 16, an absorbent core 12 arranged between said topsheet 14 and backsheet 16, and two foldable wings 24 or flaps 24 arranged at opposite sides of the sanitary napkin 10 with respect to the transversal direction T. As illustrated in FIG. 2, the sanitary napkin 10 extends in longitudinal, transversal and vertical directions L,T,V, the sanitary napkin has a longitudinal centerline, or central axis, y-y, a pair of transverse sides 30, meaning a pair of sides arranged at each longitudinal ends or longitudinal extremities, of the sanitary napkin 10, and a pair of longitudinal sides 32 extending therebetween, meaning a pair of sides arranged along each transversal ends of the sanitary napkin 10 and connecting the transverse sides 30 to one another. Similarly, the pair of transverse sides 30 connects the two longitudinal sides 32 to one another. In other words, the sanitary napkin 10 comprises two ends, or extremities, at least partially extending in the transversal direction T, *i.e.* the transversal sides 30, and two ends at least partially extending in the longitudinal direction L, *i.e.* the longitudinal sides 32. As illustrated in FIG. 2, the transversal sides 30 are arcuate whereas the longitudinal sides 32 are linear. The sanitary napkin 10 has a front edge 34 and a rear edge 36 positioned on opposite apexes of said transverse sides 30, meaning at each longitudinal extremities of the sanitary napkin 10. The sanitary napkin 10 is divided with respect to the longitudinal direction L, into three sections or portions, a front portion F, a rear portion R and a central portion C arranged between said front and rear portions F,R. The sanitary napkin 10 also comprises wings 24, or flaps 24, arranged at each transversal end of the sanitary napkin 10, the wings 24 extend transversely beyond the longitudinal sides 32 of the sanitary napkin 10. In other words, the flaps 24 are transversally, or laterally, outboard of the longitudinal centerline y-y and central portion C of the sanitary napkin 10. In other terms, each flap 24 comprises a proximal end 25 and a distal end 27 with respect to the longitudinal centerline y-y and with respect to the transversal direction T. Said proximal end 25 is connected to the sanitary napkin 10, *i.e.* connecting the sanitary napkin 10 and the wing 24 and said distal end 27 is the point furthest from the longitudinal centerline y-y. As used herein the term "central portion" C refers to the portion of the sanitary napkin 10 arranged in between the front and rear portions F, R and in between the proximal ends 25 of the flaps 24, meaning intermediate, particularly laterally intermediate of the two wings 24. In other words, the central portion C is defined by the length of the wings 24 taken in the point in which they extend from the longitudinal sides 32 of the sanitary napkin 10 in a direction parallel to the longitudinal axis y-y. In other words, the central portion C is delimited longitudinally by the front and rear portions F, R and transversally by the proximal ends 25 of the flaps 24. The wings 24 may be comprised of an integral and contiguous extension of the topsheet 14, the backsheet 16, or a laminate of both. Alternatively, the wings 24 may be made of separate and independent pieces of material joined to the longitudinal sides 32 of the sanitary napkin 10. Each wing 24 has one face generally coextensive of the topsheet 14 and a mutually opposed face generally coextensive of the backsheet 16.

As illustrated in FIG. 2, the sanitary napkin 10 comprises two regions, a main body 57 and the wings 24. The main body 57 is the region, or portion of the absorbent article 10 comprising the absorbent core 12 and the eventual ADL 18 and is oblong or oblong-shaped, meaning comprising a shape, e.g. a rectangular, that is longer than it is wide and whose corners are rounded. The wings 24 correspond to rectangular or trapezoidal extensions outboard of the main body 57, meaning outboard of the oblong shape, with respect to the transversal direction T. The wings 24 are inboard of the main body 57 with respect to the longitudinal direction L and are arranged in the central portion C of the sanitary napkin 10 with respect to the longitudinal direction L. The main body 57 can be rectangular. The wings 24, only comprising a laminate of topsheet 14 and backsheet 16, are flexible and foldable, meaning the wings 24 can be easily pivoted or rotated at least around a longitudinal axis. The topsheet 14 and the backsheet 16 are associated with one another along the periphery of the sanitary napkin 10 at the main body 57 region and at the wings 24 in bonding regions 13. The pair of longitudinal sides 32 are in reference to the main body 57.

The wings 24 preferably comprises means for attaching one face of the wing 24 to the wearer's undergarment or to the other wing 24. The wings 24 comprises at least one layer of adhesive 20. The attachment means may be pressure sensitive adhesive. If pressure sensitive adhesive is elected, it should be disposed on the face of the wing generally coextensive of the backsheet 16 so that when the wing 24 are wrapped around the crotch portion of the wearer's undergarment, the layer of adhesive will contact the outside of the wearer's undergarment. The sanitary napkin 10 for example comprises a generally rectangular patch of adhesive on each wing 24.

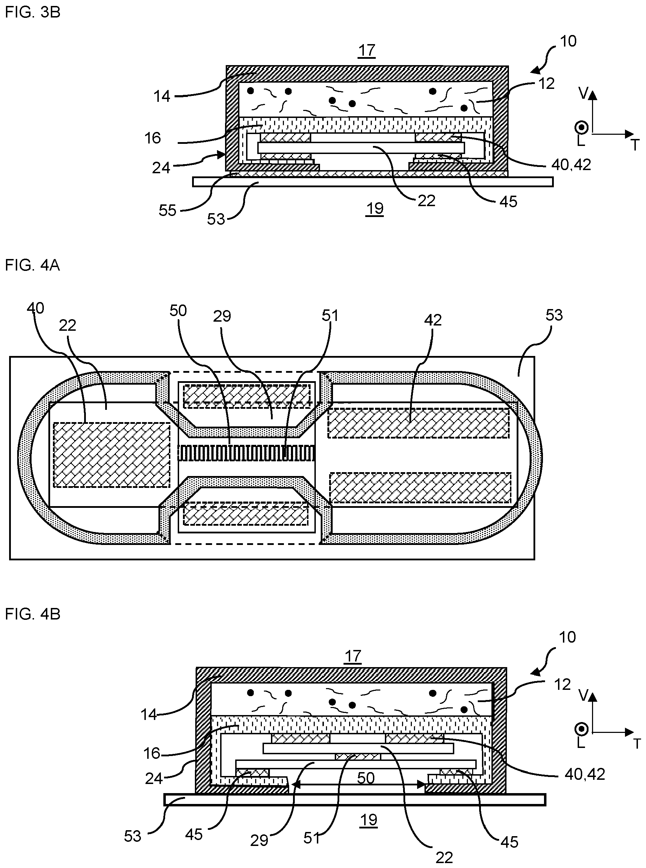

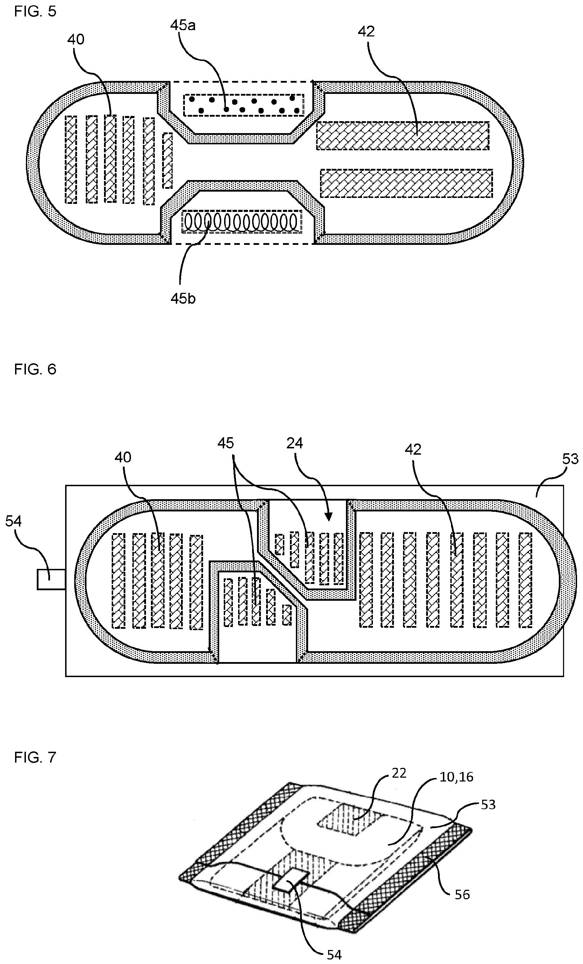

The sanitary napkin 10 comprises at least one layer of adhesive 20 to fasten the sanitary napkin 10 to an undergarment. In order to protect the layer of adhesive 20 prior to its use, the sanitary napkin 10 comprises either a release paper 22 as illustrated in FIG. 1 and/or a pouch 53, to cover directly or indirectly said at least one layer of adhesive 20 each release paper 22 and/or pouch 53 preferably comprising a layer or coating of peeling agent. Said at least one layer of adhesive 20 is arranged on one side of the backsheet 16, namely on the garment-facing side 19 of the backsheet 16, the backsheet 16 being arranged between the layer of adhesive 20 and absorbent core 12 as illustrated in FIG. 1. In other words, the at least one layer of adhesive 20 is arranged between the backsheet 16 and the release paper 22 and/or pouch 53. As illustrated in FIG. 2, the at least one layer of adhesive 20 is arranged on the backsheet 16 in a discontinuous pattern of adhesive wherein said pattern of adhesive comprises a first area of adhesive 40 arranged in the front portion F, a second area of adhesive 42 arranged in the rear portion R and a third area of adhesive 45 being arranged on each wing 24. The third area of adhesive 45 is arranged between the proximal and distal ends 25,27 of each wing 24. The pattern of adhesive comprises an adhesive-free area 46, or a gap free of adhesive, between the first area of adhesive 40 and the second area of adhesive 42 where no adhesive is present, said adhesive-free area 46 longitudinally extending on a length that is greater than or equal to the length of the third area of adhesive 45 with respect to the longitudinal direction L. In other words, the central portion C substantially does not comprise any adhesive, or there is no adhesive in the central portion C as defined hereabove. As illustrated in the drawings, the adhesive-free area 46 is in relation to the main body 57, meaning the portion of the absorbent article 10 comprising the absorbent core 12 and the eventual ADL 18 and is oblong or oblong-shaped. More specifically, the first and second areas of adhesive 40,42 are arranged on the main body 57, i.e. on the backsheet at the main body portion, with an adhesive-free area 46 arranged in between said first and second areas of adhesive 40,42 and the third area of adhesive 45 is arranged on the wings 24 which are outboard of the main body 57 with respect to the transversal direction T. The first and second areas of adhesive 40,42 can extend partially within the central portion as illustrated in FIG. 5 and FIG. 4A without departing from the invention as long as an adhesive-free area 46 is present between the first and second areas of adhesive 40,42 within the central portion C, meaning within the central portion C of the main body 57 portion of the sanitary napkin 10.

As described herein, the adhesive-free area 46 between the first and second areas of adhesive 40,42 is longitudinally extending on a length that is greater than or equal to the length of the third area of adhesive 45 with respect to the longitudinal direction L. For example, when taking the adhesive pattern as illustrated in FIG. 2, the length of each strip of adhesive in the first area of adhesive 40, is about 70 mm, the length of each strip of adhesive in the second area of adhesive 42, is about 115 mm and the length of each strip of adhesive in the third area of adhesive 45, is about 68 mm, the length of the adhesive-free area 46 is about 68 mm, e.g. comprised between 65 and 71 mm, for a sanitary napkin 10 having a total length of about 305 mm and adhesive extending on about 260 mm of that total length. In this example, the strips all have a width of about 20 mm.

The length of the first area of adhesive 40 can be more or less equal to the length of the third area of adhesive 45 and the length of the first area of adhesive 40 is lesser than the length of the second area of adhesive 42. Hence, the length of the first area of adhesive 40 can be more or less equal to the length of the adhesive-free area 46, "length" as used herein being the distance of the element, i.e. patch of adhesive or adhesive-free area or gap, in the longitudinal direction L.

According to an embodiment, the ratio of the length of the first area of adhesive 40 to the length of the third area of adhesive 45 is comprised between 0.8 and 1.2, preferably between 0.9 and 1.1. The ratio of the length of the first area of adhesive 40 to the length of the second area of adhesive 42 is comprised between 0.4 to 0.9, preferably between 0.5 and 0.7. The ratio of the length of the first area of adhesive 40 to the length of the adhesive-free area 46 is comprised between 0.8 and 1.2, preferably between 0.9 and 1.1. The ratio of the length of the adhesive-free area 46 to the length of the third area of adhesive 45 is comprised between 0.8 and 1.2, preferably between 0.9 and 1.1, more preferably about 1. Again, "length" as used herein being the distance of the element, i.e. patch of adhesive or adhesive-free area or gap, in the longitudinal direction L. Such arrangement enables that the adhesive pattern is continuously attached to the undergarment on one side or the other thereby still ensuring a good fastening of the sanitary napkin 10 onto the undergarment.

With such an adhesive pattern, it is possible to save on material, *i.e.* use less adhesive. It is also possible with this pattern of adhesive to fold the wings 24 outward, meaning toward the backsheet 16 since the adhesive normally present in the central portion C cannot hinder the unfolding of the wings 24. As illustrated in FIG. 3A, with such configuration, the wings can be folded onto the backsheet without the layer of adhesive 20 present on the backsheet 16, specifically the layer of adhesive 20 arranged on the backsheet 16 in the front and rear portions F,R, adhering to the wings 24. Of course, it is also possible to fold the wings 24 inward toward the topsheet 14 and still save on material. In other words, comprising a gap meaning an area free of adhesive, between the first and second areas of adhesive 40,42, offers the possibility to fold the wings onto the topsheet 14 or onto the backsheet 16, said adhesive-free area 46 longitudinally extending on a length that is greater than or equal to the length of the third area of adhesive 45 with respect to the longitudinal direction L. Preferably, the adhesive-free area46 is longitudinally extending on a length that is more or less equal to the length of the third area of adhesive 45 with respect to the longitudinal direction L, such arrangement enables that the adhesive pattern is continuously attached to the undergarment on one side or the other thereby still ensuring a good fastening of the sanitary napkin 10 onto the undergarment.

In order to protect the third area of adhesive 45 of the pattern of the layer of adhesive 20 and avoid the third areas of adhesive 45 from sticking to the backsheet 16, the sanitary napkin 10 can comprise a second release paper 29 covering the adhesive arranged on the wings 24, *i.e.* the third areas of adhesive 45, as illustrated in FIG.4A and 4B. The sanitary napkin 10 can comprise two second release paper 29, one for covering each third area of adhesive 45, or one single second release paper 29 covering simultaneously both third areas of adhesive 45. The second release paper(s) 29 is similar to the first release paper 22 in material, meaning said second release paper 29 comprises a siliconized paper or a paper layer coated with a silicone derivative.

Alternatively, it is possible to arrange a single release paper 22 covering all three areas of adhesive 40,42,45 as illustrated in FIG. 3A and FIG. 3B where the first and second areas of adhesive 40,42 are on one side of the release paper 22, namely the body-facing side 17, and the third area of adhesive is arranged on the opposite side of the release paper 22, namely the garment-facing side 19. In other words, with reference to FIG. 3B, the release paper 22 comprises a body-facing side 17 and a garment-facing side 19. The first and second areas of adhesive 40,42 are covered, or protected by the body-facing side 17 of the release paper 22, meaning that the first and second areas of adhesive 40,42 are arranged between the body-facing side 17 of the release paper 22 and the backsheet 16, or main body region, whereas the third area of adhesive 45 is arranged between the garment-facing side 19 of the release paper 22 and the wings 24, this way it is possible to save on release paper and have only one single sheet of release paper covering the adhesive on both the wings 24 and the main body. With such embodiment, it is possible to remove the release paper 22 and uncover the layer of adhesive 20 and unfold the wings 24 in one linear movement for example by pulling on the release paper 22 in one direction from front to back. As illustrated in FIG. 3B, an optional layer of adhesive 55 can be arranged on the pouch 53 so that the sanitary napkin 1 can adhere onto said pouch 53, namely, the main body and wings can adhere onto said pouch 53. Alternatively, as illustrated in FIG. 4B, the sanitary napkin 10 can be arranged within the pouch 53 with no additional layer of adhesive in between.

As discussed above and as illustrated in FIG. 2, the at least one layer of adhesive 20 is applied in a pattern, *i.e.* a pattern of adhesive, said pattern of adhesive comprising three areas of adhesive 40,42,45 and an adhesive-free area 46 extending transversally between the two wings 24, more precisely between the two proximal ends 25 of the wings 24 and longitudinally between the first and second areas of adhesive 40,42. The adhesive can be applied in one bloc, e.g. FIG. 4A - first area of adhesive 40, and/or in stripes, e.g. FIG. 4A - second area of adhesive 42, and/or in dots, e.g. FIG. 5 - third area of adhesive 45a, and/or in swirls e.g. FIG. 5 - third area of adhesive 45a. The invention is not limited to these manners of applying adhesive and can be applied in stars, triangles, *etc.* The adhesive can be applied in one, two, three or more patches, e.g. blocs, squares, stripes, dots, swirl, in a given area. For example in FIG. 3A, the adhesive is applied in the front region F, *i.e.* the first area of adhesive 40, in two stripes longitudinally elongated on each side of the centerline y-y.

According to an embodiment such as illustrated in FIG. 3A, the first area of adhesive 40 and second area of adhesive 42 are aligned on the same longitudinal axis. For example, the first area of adhesive 40 and second area of adhesive 42 each comprises at least one strip extending longitudinally and aligned on a same longitudinal axis, such as the centerline y-y. As illustrated in FIG. 2, the first area of adhesive 40 and the second area of adhesive 42 each comprises two longitudinally oriented strips of adhesive with one strip on each side of the longitudinal centerline y-y and each aligned on a respective longitudinal axis. In other words, each first and second areas of adhesive 40,42 comprises a pair of strips of adhesive extending longitudinally. For example, the strip of adhesive extending longitudinally in the rear portion R extends on a same axis as, i.e. is aligned with, a strip of adhesive extending longitudinally in the front portion, e.g. the two strips of adhesive 40,42 in the front and rear portions F,R on one same side of the centerline y-y extend along a same longitudinal axis. Such arrangement simplifies the process as it requires a single glue applicator with slots coating.

The present disclosure is not limited to longitudinally aligned areas of adhesive. For example the first and/or second areas of adhesive 40,42 can comprise transversally extending strips with the pattern of adhesive still presenting a adhesive-free area 46 in the central portion C as illustrated in FIG. 5 or FIG. 6. Such arrangement also simplifies the process as it requires a single glue applicator with slots coating. The first area of adhesive 40 and second area of adhesive 42 can comprise different arrangement of adhesive as illustrated in FIG. 5 where the first area of adhesive 40 comprises a plurality of strips of adhesive extending transversally and the second area of adhesive 42 comprises two strips of adhesive extending longitudinally. In FIG. 4A, the first area of adhesive 40 comprises one bloc of adhesive whereas the second area of adhesive 42 comprises two longitudinally elongated stripes of adhesive. The longitudinally or transversally extending strips can be of the same dimensions (width, length) or of different dimensions. In the example illustrated in FIG. 5, a third area of adhesive 45a arranged on a wing 24 comprises dots of adhesive arranged in a rectangular area extending longitudinally whereas the other third area 45b, arranged on the opposite wing 24, comprises swirls of adhesive arranged in a rectangular area extending longitudinally. Preferably, from a process point of view, it is more practical if the adhesive is applied in a identical manner in all three areas 40,42,45 or at least in a identical manner in both the first and second areas of adhesive 40,42. For example, the first and second areas 42 each comprises transversally extending stripes and the third areas 45 comprises longitudinally extending stripes. For example, all three areas of adhesive 40,42,45, comprise longitudinally extending stripes. For example, all three areas of adhesive 40,42,45 comprise transversally extending stripes with gaps between the stripes being of equal or different lengths.

According to an embodiment, the third area of adhesive 45 is skewed or shifted with first and second areas of adhesive 40, 42 when all areas of adhesive area projected on the longitudinal centerline y-y. In other words, the first, second and third areas of adhesive 40,42,45 do not overlap when projected on a longitudinal axis.

According to an embodiment as illustrated in FIG. 3A and FIG. 3B, when the wings 24 are folded onto the main body, the first second and third area of adhesive 40,42,45 are all aligned on a same axis extending in the longitudinal direction L. According to this embodiment, the wings 24 are in close proximity with one another, meaning they can touch, overlap or at least cover more than 80 % of the distance between the two proximal point 25. According to an embodiment as illustrated in FIG. 4A and FIG4B, when the wings 24 are folded onto the main body, meaning the wings 24 are folded towards the backsheet 1, the first, second and third areas of adhesive 40,42,45 all extend in the longitudinal direction L along parallel longitudinal axis that are skewed, or shifted, with respect to the transversal axis T. According to that same illustration, it is possible to arrange a gap 50, with respect to the transversal direction T, between the wings 24 when folded over, meaning the wings 24 do not touch, or overlay, one another when folded over. In other words, the wings 24 are dimensioned so that they cannot overlap. With such arrangement, it is possible to arrange an additional layer of adhesive 51, or fourth area of adhesive 51, arranged between the first and second release papers 22,29 so that when pulling on the first release paper 22, it also removes the second release paper 29 thereby uncovering the first, second and third areas of adhesive in one movement. In other words, the peeling of the first release paper 22 induces or causes the removal of the second release paper 29 given that both release papers 22,29 are connected by an additional layer of adhesive 51. Such arrangement enables to uncover all adhesive areas 40, 42, 45 in one linear movement. The fourth adhesive layer 51 preferably presents a better adhesion strength than the other adhesive areas 40,42,45 to ensure a better removal of the second release paper 29. For example, the first, second, third areas of adhesive 40,42,45 each comprises pressure sensitive adhesive whereas the fourth area of adhesive 51 comprises hot melt adhesive.

According to an embodiment as illustrated in FIG. 6, the wings 24 can be asymmetrical with respect to the longitudinal centerline y-y. Here the wings 24 are rectangle with one corner snipped, i.e. a pentagon with three right angles. The adhesive pattern still comprises a first, second and third areas of adhesive 40,42,45 as described hereabove with an adhesive-free area 46 between the first and second areas of adhesive 40,42. The adhesive is applied here in all three areas 40,42,45 in transversally extending stripes with the third areas comprising here some stripes of different heights to match the outline of the wings 24.

Sanitary napkins 10 are sold in a folded state, either in a three-time fold, meaning comprising two fold lines, or in a two-time fold, meaning comprising one fold line, to fit more articles in a rectangular carboard box or plastic bag. The sanitary napkin 10 extends in longitudinal, transversal and vertical directions L, T, V in a folded or unfolded configuration or state. The sanitary napkin 10 comprises a wrapper 53, also called pouch 53, as shown in FIG. 7 to keep the sanitary napkin 10 in a folded state. The sanitary napkin 10 can comprise both a release paper 22 and a pouch 53 as illustrated in FIG. 7, or the sanitary napkin 10 can comprise only a pouch 53 acting as a clamping mean to keep the sanitary napkin 10 folded and as a covering mean to cover the at least one layer of adhesive 20. In other words, the release paper 22 is removed from the absorbent article. The same principle described above for the release paper applies when using the pouch as a release liner. For example the sanitary napkin 10 can comprise a pouch 53 to cover the first and second areas of adhesive 40,42 and a second release paper 29 to cover the third area of adhesive 45.

As shown in FIG. 6, the sanitary napkin 10 comprises a pouch, or a wrapper, 53 that has a generally rectangular configuration with an inner surface on which the absorbent article 10 is arranged and an outer surface on which a closing tape 54 is arranged. In other terms, the inner surface is arranged on the body-facing side 17 and the outer surface is arranged on the garment-facing side 19. The closing tape 54 is provided with a grasping section that is free of adhesive so that the user can grasp the closing tape 54. The closing tape 54 also comprises an attaching section that comprises adhesive so that the closing tape 54 can adhere to the outer surface of the wrapper 53 especially when it is used as a disposal wrap. When used as a disposal-wrap, the closing tape 54 can be used to contain the soiled article within the pouch 53 by attaching two ends of the wrapper 53 with the soiled article within said wrapper 53.

Although the closing tape 54 is arranged to maintain the pouch 53 in a closed configuration, the absorbent article 10 being folded, will have a natural tendency to revert to its unfolded configuration and the closing tape 54 is usually not sufficient to maintain the absorbent article 10 in a folded configuration and the wrapper 53 closed. To that end, the wrapper 53 preferably comprises a layer of sealing agent material and/or undergoes a sealing treatment to obtain sealed portions 56. The sealing treatment may be carried out using any suitable means known without any particular limitation. For example, the sealing treatment can be carried out either by applying, or coating, a sealing agent on an area of the inner surface of the wrapper 53, folding the wrapper 53 and absorbent article 10 and then applying temperature and/or pressure to this agent material to achieve the sealing. For example and not limited to, the sealing treatment can be carried out by thermo-sealing (applying heat to a heat-sealable material), ultrasonic sealing (applying ultrasounds, thus heat, to a heat-sealable material), gluing (applying an adhesive material, preferably weak such as a non-structural adhesive, and then applying pressure). The wrapper 53 can be made out of a plastic film, preferably a heat-sealable plastic material such as polyethylene, or the wrapper can be made out of paper with a layer of heat-sealable material at its periphery.

The wrapper 53 according to an embodiment comprises cellulosic fibers or in other terms, the wrapper 53 preferably contains a sustainable material and more specifically cellulose pulp. Preferably, the wrapper 53 comprises paper material, or cellulosic fibres, coming from natural sources for example and not limited to wood (softwood or hardwood), cotton or hemp. A nonwoven fabric of cellulosic fibers, *e.g*. paper, is less flexible and rougher than a plastic film. To that intent, the wrapper 53 comprises a layer consisting essentially of cellulosic fibers, e.g. a paper layer, comprising a basis weight comprised between 10 gsm and 40 gsm, preferably between 15 gsm and 35 gsm, more preferably between 20 gsm and 30 gsm. More preferably, the layer of cellulosic faber of the wrapper 53 consists essentially of paper material. By using a pulp such as paper or any other natural cellulose fibers corresponding to an environment friendly material, the environmental impact of the packaging is lowered. By comprising a basis weight comprised between 10 gsm and 40 gsm, the pouch 53 retains the functionalities of the plastic film conventionally used. The basis weight is sufficiently high to ensure that the wrapper 53 is robust enough to contain an absorbent article 10 and not tear easily. The basis weight is sufficiently low to ensure that the wrapper is flexible enough and can be easily folded. Another parameter of the layer of cellulosic fibers is its thickness, specifically, the layer of cellulosic fibers 33 has a thickness between 5 µm and 50 µm. Again, this thickness is sufficiently high to ensure that the wrapper 53 is robust enough to contain an absorbent article 10 and not tear easily. This thickness is sufficiently low enough to ensure that the wrapper 53 is flexible and can be easily folded. Thickness of a layer can be determine using any standard technics such as measuring the wrapper or paper layer with a caliper preferably a micrometer caliper gauge and the basis weight of the layer of cellulosic fibers is preferably measured according to the ISO 536 standard or any standard method.

Given that cellulosic fibers, in particular paper, and plastic material are materials that can easily be printed on, the wrapper 53 can be provided with graphic elements that are printed, by common techniques such as flexographic printing, gravure printing or inkjet printing, onto the outer surface and/or inner surface of the wrapper 53.

The layer of cellulosic fibers can impede the good peeling of the adhesive layer 20 and/or release paper 22,29 and thus of the absorbent article 10. To that end, the wrapper 53 preferably comprises a layer of peeling agent or undergoes a peeling treatment. The peeling treatment may be carried out using any suitable means known without any particular limitation. For example, the peeling treatment can be carried out either by applying, or coating, peeling agent material onto an area of the inner surface of the wrapper 53 or by adhering a peelable tape or a peelable paper, to an area of the inner surface of the wrapper 53.

The peeling agent or the peeling treatment applied to the release paper 22,29, peelable tape 54 or the peelable wrapper 53 thereof are preferably selected from and not limited to silicon resin series, fluororesin series, octadecilisocyanate series or the like. It is particularly preferable that the peeling treatment is carried out by applying a coat of silicon resin series as the peeling agent material. The peeling agent is dried by heating or by irradiating ultraviolet rays or the like so as to be polymerized, or by spraying it, to thereby form a thin coat or thin layer of peeling agent. Using a thin coat of silicone as the peeling agent is preferable since silicone corresponds to a material that has heat-resistant properties, thus more stable in industrial processes, as well as antimicrobial properties which is better to ensure the protection of the absorbent article from contamination prior to use. The silicone has other properties such as water repellent and is greaseproof. The wrapper 53 comprises a layer of peeling agent comprising a basis weight comprised between 0,3 gsm and 3 gsm, preferably between 0,5 gsm and 1 gsm.

For these embodiments comprising a sealing treatment as described above, i.e. implying the use of a sealing agent such as an adhesive or a heat-sealable material, it is preferable that the layer of sealing agent comprises a non-petroleum derived material as to lower the environmental impact of the absorbent article. The layer of sealing agent comprises for example and not limited to polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL). The layer of sealing agent 34 can comprise a polymer material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The sealing agent preferably comes from a sustainable source, or bio-based or bioplastic, and is biodegradable and/or compostable. The layer of sealing agent has a basis weight comprised between 1 gsm and 20 gsm, preferably between 3 gsm and 15 gsm, more preferably between 5 gsm and 10 gsm.

It is supposed that the present invention is not restricted to any form of realization described previously and that some modifications can be added to the presented example of fabrication without reappraisal of the appended claims.

## Claims

1. Sanitary napkin (10) comprising :
- a topsheet (14),
- a backsheet (16),
- an absorbent core (12) arranged between said topsheet (14) and backsheet (16),
- two wings (24),
the sanitary napkin (10) extending in longitudinal, transversal and vertical directions (L,T,V), the sanitary napkin (1) being divided with respect to the longitudinal direction, into a front portion (F), a rear portion (R) and a central portion (C) arranged between said front and rear portions, the central portion (C) being defined by the length of the wings (24) taken in the point in which they extend from the longitudinal sides (32) of the sanitary napkin (10) in a direction parallel to the longitudinal axis (y-y),
wherein a layer of adhesive (20) is arranged on the side of the backsheet (16) opposite to the absorbent core (12),
**characterized in that** said layer of adhesive (20) is arranged on the backsheet (16) in a discontinuous pattern, said pattern of adhesive comprising:
- a first area of adhesive (40) arranged in the front portion (F),
- a second area of adhesive (42) arranged in the rear portion (R);
- a third area of adhesive (45) being arranged on the wings (24);
wherein said pattern of adhesive comprises an adhesive-free area (46) between the first area of adhesive (40) and the second area of adhesive (42), said adhesive-free area (46) longitudinally extending on a length that is greater than or equal to the length of the third area of adhesive (45) with respect to the longitudinal direction (L).

2. Sanitary napkin (10) according to claim 1, wherein said adhesive-free area (46) extends transversally between the two wings (24).

3. Sanitary napkin (10) according to any of the preceding claims, wherein the first area of adhesive (40) and second area of adhesive (42) are aligned on one same longitudinal axis.

4. Sanitary napkin (10) according to any of the preceding claims, wherein the third area of adhesive (45) is longitudinally skewed from the first and second areas of adhesive (40,42) when projected on the longitudinal axis.

5. Sanitary napkin (10) according to any of the preceding claims, wherein when the wings (24) are folded onto the backsheet (16) or topsheet (12), the first, second and third areas of adhesive (40,42,45) are aligned with respect to the longitudinal axis.

6. Sanitary napkin (10) according to any of the preceding claims, wherein the first, second and/or third areas of adhesive (40,42,45) comprise strips of adhesive, said strips extending transversally and/or longitudinally.

7. Sanitary napkin (10) according to any of the preceding claims, wherein a first release paper (22,29) and/or a pouch (53) is arranged on one side of the layer of adhesive (20) such that the layer of adhesive (20) is arranged between the backsheet (16) and the release paper (22,29) and/or pouch (53).

8. Sanitary napkin (10) according to claim 7, wherein a single release paper (22) simultaneously covers all three areas of adhesive (40,42,45), first and second areas of adhesive (40,42) being covered by a first side of the release paper (22) and the third area of adhesive (45) being covered by the opposite side of the release paper (22).

9. Sanitary napkin (10) according to claim 7, wherein the sanitary napkin (10) comprises a second release paper (29) covering the third areas of adhesive (45), the wings (24) being separated from one another by a gap (50) with respect to the transversal direction T when folded over the backsheet (16), an additional layer of adhesive (51) being arranged between the first and second release papers (22,29) within said gap (50).

10. Method suitable for packaging a sanitary napkin for obtaining an assembly according to claim 1 to 9, the method comprising
- providing a sanitary napkin comprising a topsheet, backsheet and an absorbent core arranged in between,
- applying a layer of adhesive according to a discontinuous pattern of adhesive wherein said pattern of adhesive comprises:
- a first area of adhesive (40) arranged in the front portion (F),
- a second area of adhesive (42) arranged in the rear portion (R);
- a third area of adhesive (45) being arranged on the wings (24);
wherein the pattern of adhesive comprises an adhesive-free area (46) between the first area of adhesive (40) and the second area of adhesive (42), said adhesive-free area (46) extending on a greater length than the third area of adhesive (45) with respect to the longitudinal direction (L).
